# EUROPÄISCHE PATENTSCHRIFT

(11) **EP 2 174 102 B1**
(45) Veröffentlichungstag und Bekanntmachung des Hinweises auf die Patenterteilung: **12.09.2018**
(21) Anmeldenummer: 08774949.5
(22) Anmeldetag: 09.07.2008
(51) Int. Cl.: G01G 21/22, A61B 5/00, A61B 5/053, G01G 19/44

(54) **PERSONENWAAGE**
PERSONAL SCALES
PÈSE-PERSONNE

(30) Priorität: 26.07.2007 DE 102007034980
(43) Veröffentlichungstag der Anmeldung: 14.04.2010
(73) Patentinhaber: BSH Hausgeräte GmbH, 81739 München (DE)
(72) Erfinder: KLEMP, Eric, 33106 Paderborn (DE); WEISBARTH, Yvonne Janet, 82031 Grünwald (DE)
(86) Internationale Anmeldenummer: PCT/EP2008/058945
(87) Internationale Veröffentlichungsnummer: WO 2009/013138

(56) Entgegenhaltungen:
- EP-A- 1 201 187
- EP-A1- 1 018 639
- WO-A-98/33045
- US-A- 4 043 413
- US-A1- 2004 129 463
- US-A1- 2007 167 286
- US-B1- 6 864 436

## Beschreibung

Die Erfindung betrifft eine Personenwaage umfassend wenigstens eine Basiseinheit, ein in der Basiseinheit integrierbares Kraftmesselement, eine auf das Kraftmesselement wirkende Wiegeplatte, sowie eine Anzeige zum Anzeigen zumindest des von dem Kraftmesselement gemessenen Gewichts.

Personenwaagen sind in einer Vielzahl im Stand der Technik bekannt. So ist aus der WO 98/33045 A eine modular aufgebaute Personenwage bekannt, deren Wiegeplatte getrennt von einer Basiseinheit als Modul gefertigt und/oder transportiert werden kann. Dabei werden die Wiegeplatte und das Basismodul an einem separaten Standort durch Stoffschluss oder Formschluss zu einer konventionellen Personenwaage zusammengefügt. Aus der EP 1 018 639 A1 ist eine Waage bekannt, deren Wiegeplatte mit Schrauben auf einer Basiseinheit fixiert ist. Dazu offenbart die US 4 043 413 A eine Personenwaage die aus einer Bodenplatte, einer darauf aufliegenden Wiegeeinheit und einer auf der Wiegeeinheit aufgelegten Wiegeplatte besteht. Diese Waage besteht dabei aus drei möglichst leichten und einfachen Modulen um z.B. zum Transport im Reisegepäck auf sehr einfache Weise zerlegbar zu sein. Aus der US 6 864 436 B1 ist zudem eine Personenwaage bekannt, die aus einem flächenbündig in den Fußboden einlegbaren Sensorpad und einer damit drahtlos verbundenen Anzeigeeinrichtung besteht. Die US 2007/167286 A1 offenbart eine herkömmlich aufgebaute Personenwaage, die Daten zur Speicherung in Benutzerprofilen erfasst und diese Profile durch eine biometrische Zugangskontrolle sichert. Dazu ist aus der US 2004/129463 A1 eine konventionell aufgebaute Personenwaage mit Speichereinrichtung für die Gewichtshistorie bekannt, deren Benutzungsoberfläche eine antibakterielle Beschichtung aufweist. Die in EP 1 201 187 A offenbarte konventionell aufgebaute Personenwaage ist dazu eingerichtet neben dem Gewicht eines Anwenders weitere Informationen wie z.B. einen Body Mass Index zu erfassen, zu verarbeiten und auf einer Anzeigeeinrichtung in einfach verständlicher Weise anzugeben.

Dabei weisen die im Stand der Technik verfügbaren Personenwaagen eine Reihe von Nachteilen auf. Wird die Wiegeplatte einer Waage beschädigt oder erheblich verunreinigt, so bleibt dem Anwender nur die Möglichkeit, die gesamte Waage auszutauschen. Wünscht der Anwender eine Veränderung des äußeren Erscheinungsbildes der Waage, so ist er ebenfalls darauf angewiesen, die komplette Waage auszutauschen. Darüber hinaus ist es bei einer Personenwaage, die in der Regel mit bloßen Füßen betreten wird, wünschenswert, dass sich diese leicht und effektiv reinigen lässt. Dies gilt insbesondre für Personenwaagen in Mehr-Personen-Haushalten, in denen Personenwaagen von mehreren Personen genutzt werden.

Somit ist es Aufgabe der vorliegenden Erfindung, eine Personenwaage zur Verfügung zu stellen, die sich auf sichere Weise leicht reinigen lässt, und sich insbesondere auf einfache und sichere Weise nach den Wünschen eines individuellen Anwenders umgestalten lässt.

Gelöst wird diese Aufgabe durch eine Personenwaage mit den Merkmalen des Patentanspruches 1. Vorteilhafte Aus- und Weiterbildungen der Erfindung, welche einzeln oder in Kombination miteinander eingesetzt werden können, sind Gegenstand der Unteransprüche.

Die erfindungsgemäße Personenwaage baut auf gattungsgemäßen Personenwaagen dadurch auf, dass eine Halterung vorgesehen ist, mittels derer die Wiegeplatte durch einen Anwender entnehmbar und gegen eine andere Wiegeplatte austauschbar ist. Dadurch wird gegenüber dem Stand der Technik eine erhebliche Verbesserung der Personenwaage erzielt. Die Wiegeplatte wird erfindungsgemäß über eine verschiebbare Fixierung und/oder eine axial fixierende Arretierung fixiert. Dies ermöglicht auf der einen Seite eine sichere Befestigung der Wiegeplatte mit der Basiseinheit, so dass die Gewichtsdaten zuverlässig und sicher ermittelt werden können und auf der anderen Seite gleichzeitig, dass die Personenwaage gut transportiert werden kann, ohne dass die Wiegeplatte sich versehentlich aus der Arretierung löst. Gleichzeitig ermöglicht diese Befestigung aber auch einen einfachen und für den Anwender wenig aufwändigen Austausch der Wiegeplatte gegen eine andere Wiegeplatte. Indem die Wiegeplatte der Personenwaage entnommen und so bleibt dem Anwender nur die Möglichkeit, die gesamte Waage auszutauschen. Wünscht der Anwender eine Veränderung des äußeren Erscheinungsbildes der Waage, so ist er ebenfalls darauf angewiesen, die komplette Waage auszutauschen. Darüber hinaus ist es bei einer Personenwaage, die in der Regel mit bloßen Füßen betreten wird, wünschenswert, dass sich diese leicht und effektiv reinigen lässt. Dies gilt insbesondre für Personenwaagen in Mehr-Personen-Haushalten, in denen Personenwaagen von mehreren Personen genutzt werden.

Somit ist es Aufgabe der vorliegenden Erfindung, eine Personenwaage zur Verfügung zu stellen, die sich auf sichere Weise leicht reinigen lässt, und sich insbesondere auf einfache und sichere Weise nach den Wünschen eines individuellen Anwenders umgestalten lässt.

Gelöst wird diese Aufgabe durch eine Personenwaage mit den Merkmalen des Patentanspruches 1. Vorteilhafte Aus- und Weiterbildungen der Erfindung, welche einzeln oder in Kombination miteinander eingesetzt werden können, sind Gegenstand der Unteransprüche.

Die erfindungsgemäße Personenwaage baut auf gattungsgemäßen Personenwaagen dadurch auf, dass eine Halterung vorgesehen ist, mittels derer die Wiegeplatte durch einen Anwender entnehmbar und gegen eine andere Wiegeplatte austauschbar ist. Dadurch wird gegenüber dem Stand der Technik eine erhebliche Verbesserung der Personenwaage erzielt. Dies ermöglicht auf der einen Seite eine sichere Befestigung der Wiegeplatte mit der Basiseinheit, so dass die Gewichtsdaten zuverlässig und sicher ermittelt werden können und auf der anderen Seite gleichzeitig, dass die Personenwaage gut transportiert werden kann, ohne dass die Wiegeplatte sich versehentlich aus der Arretierung löst. Gleichzeitig ermöglicht diese Befestigung aber auch einen einfachen und für den Anwender wenig aufwändigen Austausch der Wiegeplatte gegen eine andere Wiegeplatte. Indem die Wiegeplatte der Personenwaage entnommen und unabhängig von der Basiseinheit gereinigt werden kann, werden die Reinigungsmöglichkeiten gegenüber dem Stand der Technik erheblich verbessert. Wird eine Waage von mehreren Personen genutzt, so ist es vorteilhaft möglich, für jede Person je nach Geschmack eine andere Wiegeplatte einzusetzen. Dies bedeutet sowohl hinsichtlich der optischen Gestaltungsmöglichkeiten als auch der hygienischen Situation der Waage eine entscheidende Verbesserung.

Erfindungsgemäß bevorzugt sind wenigstens zwei, vorzugsweise vier, Basiseinheiten vorgesehen, welche vorteilhaft verteilt angeordnet auch hohe Gewichte aufzunehmen vermögen.

Je nach Ausgestaltung der Waage hat es sich bewährt, wenigstens eine Tragstruktur zur Halterung der Basiseinheit/en vorzusehen. Diese Tragstruktur gewährt verbesserte gestalterische Freiheiten bei gleichzeitig hoher Stabilität und Belastbarkeit.

Dabei ist es von besonderem Vorteil, wenn in einer bevorzugten Ausführungsform die Personenwaage eine Speichereinheit aufweist, die Informationen speichert, mittels derer eine definierte Wiegeplatte einer definierten Person zugeordnet werden kann. Hierdurch kann das Risiko einer Verwechslung von Daten vermindert werden.

Es hat sich zudem als vorteilhaft erwiesen, wenn die Waage eine elektronische Speichereinheit zum Speichern von Benutzerdaten, insbesondre Gewichtsdaten, aufweist. Wird von dem Anwender z.B. eine gezielte, auf mehrere Tage oder Wochen angelegte Maßnahme zur Gewichtsreduktion unternommen, ist es für ihn von großem Vorteil, wenn er mit Hilfe der Speichereinheit Gewichtsdaten zu verschiedenen Zeitpunkten abspeichern kann, und bei Bedarf auf einfache Weise einen Verlauf seiner Gewichtsentwicklung abrufen kann. Besonders vorteilhaft ist es, wenn die elektronische Speichereinheit Teil der Wiegeplatte ist. So können z.B. in einem Haushalt einer vierköpfigen Familie vier Wiegeplatten mit jeweils eigener Speichereinheit eingesetzt werden, wobei jeweils eine Wiegeplatte bzw. eine Speichereinheit einer Person zugeordnet wird. Auf der Speichereinheit lassen sich dann Daten der Person, der die betreffende Wiegeplatte zugeordnet wurde, speichern. Dabei ist es von besonderem Vorteil, wenn die Speichereinheit Informationen speichert, mittels derer eine definierte Wiegeplatte einer definierten Person so zugeordnet werden kann, dass eine Verwechslung ausgeschlossen werden kann. Dies kann in einer bevorzugten Ausführungsform dadurch erfolgen, dass dem Anwender mittels eines passwords Zugang zu einem ihm zugeordneten Informationssegment verschafft wird.

Von besonderem Vorteil ist es dabei, wenn die Speichereinheit eine Datenschnittstelle zur Datenübertragung an einen zugeordneten Computer aufweist. Die übertragenen Daten können dann im Computer intensiv bearbeitet und ausgewertet werden. In einem weiteren Schritt kann der Computer eine Gewichtsprognose entwerfen, anhand derer der Anwender eine Information erhält, in welchem Zeitraum sich sein Gewicht wie entwickeln sollte. Über die Datenschnittstelle kann diese Gewichtsprognose wieder in die Speichereinheit übertragen werden. Die Personenwaage kann dann dem Anwender anzeigen, ob sich seine Gewichtsentwicklung im Rahmen der vom Computer entworfenen Prognose bewegt bzw. davon abweicht.

Von besonderem Vorteil ist es, wenn die Datenübertragung zum Computer mittels Funk und/oder Infrarotübertragung und/oder Übertragungskabel erfolgt.

Es hat sich als besonders vorteilhaft erwiesen, wenn die Personenwaage eine biometrische Zugangskontrolle aufweist, die sicherstellt, dass erst nach Erfüllen eines oder mehrerer Zugangskriterien für einen Anwender ein definierter Speicherbereich und/oder ein definiertes Programm der Personenwaage zugänglich ist. Eine biometrische Zugangskontrolle stellt sicher, dass ein Anwender nicht irrtümlich in einen Speicherbereich gelangt, der einer anderen Person zugeordnet war.

Besonders vorteilhaft ist es dabei, wenn die biometrische Zugangskontrolle über einen Vergleich der Stimme und/oder der Iris und/oder des Fuß- und/oder Fingerabdruckes erfolgt. Diese Zugangskontrolle erfolgt sehr schnell und einfach, wobei eine Verwechslungsgefahr fast völlig ausgeschlossen werden kann.

In einer bevorzugten Ausführungsform weist die Wiegeplatte ein Display auf. Dabei ist es von besonderem Vorteil, wenn das Display ein Tastenfeld aufweist und/oder auswechselbar ist.

Von Vorteil ist es auch, wenn die Wiegeplatte wenigstens ein Wechselbauteil umfasst, welches aus der Wiegeplatte entnehmbar ausgebildet ist.

Von besonderem Vorteil ist es dabei, wenn das Wechselbauteil ein Mikrochip und/oder die Speichereinheit ist. Auf einem Mikrochip lassen sich auf kleinstem Raum große Datenmengen speichern.

In einer bevorzugten Ausführungsform weist die Wiegeplatte eine antibakterielle Beschichtung auf. Diese Beschichtung kann auf einer oder auf beiden Seiten der Wiegeplatte ausgeführt werden. Eine antibakterielle Beschichtung der Wiegeplatte ist besonders sinnvoll, da die Wiegeplatte in der Regel mit nackten Füßen betreten wird, und häufig wechselweise von verschiedenen Personen genutzt wird. Es kann unter Umständen erforderlich sein, dass die antibakterielle Beschichtung erneuert oder verändert werden muss. Dies kann in einer einfachen und zweckmäßigen Weise dadurch erfolgen, dass die Wiegeplatte entnommen, entsprechend behandelt, und wieder eingesetzt wird.

Von besonderem Vorteil ist es dabei, wenn die Waage eine Körperfettwaage ist.

Die erfindungsgemäße Personenwaage weist gegenüber den herkömmlichen Personenwaagen erheblich verbesserte Gestaltungsmöglichkeiten auf und ist darüber hinaus auch in hygienischer Hinsicht vorteilhaft.

Weitere Vorteile und Ausgestaltungen der Erfindung werden im Folgenden anhand mehrerer Ausführungsbeispiels, auf welche die vorliegende Erfindung jedoch nicht beschränkt ist, sowie unter Bezugnahme auf die beiliegenden Zeichnungen erläutert.

Darin zeigen schematisch:
- Fig. 1:: eine schematische Darstellung einer Personenwaage;
- Fig. 2:: eine Darstellung einer ersten Ausführungsform der Befestigung der Wiegeplatte; sowie
- Fig. 3:: eine Darstellung einer zweiten Ausführungsform der Befestigung der Wiegeplatte.

Bei der nachfolgenden Beschreibung der bevorzugten Ausführungsformen der vorliegenden Erfindung bezeichnen gleiche Bezugszeichen gleiche oder vergleichbare Komponenten.

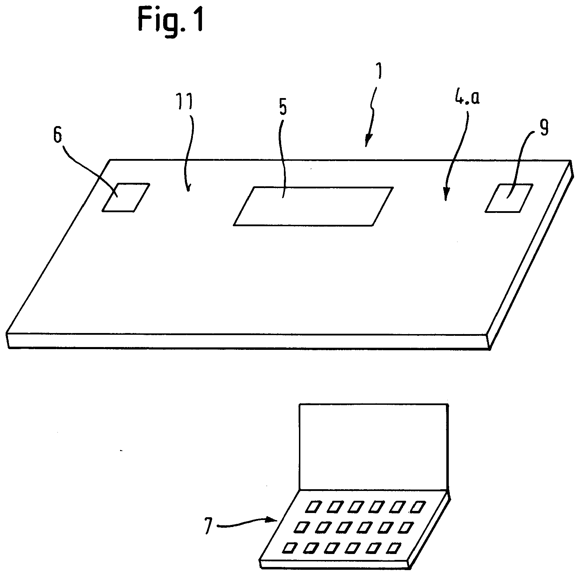

Fig. 1 zeigt in einer schematischen Darstellung eine Personenwaage 1. Diese Personenwaage 1 weist eine Wiegeplatte 4.a mit einer Anzeige 5 auf. Darüber hinaus weist die Personenwaage 1 auf der dem Benutzer zugewandten Seite 11 eine Speichereinheit 6 auf. Diese ist als Teil der Wiegeplatte 4.a ausgebildet. Die Speichereinheit 6 weist eine Datenschnittstelle zur Datenübertragung an einen zugeordneten Computer 7 auf. Darüber hinaus weist die Wiegeplatte 2 ein Wechselbauteil 9 auf. Dieses Wechselbauteil 9 ist ein Mikrochip.

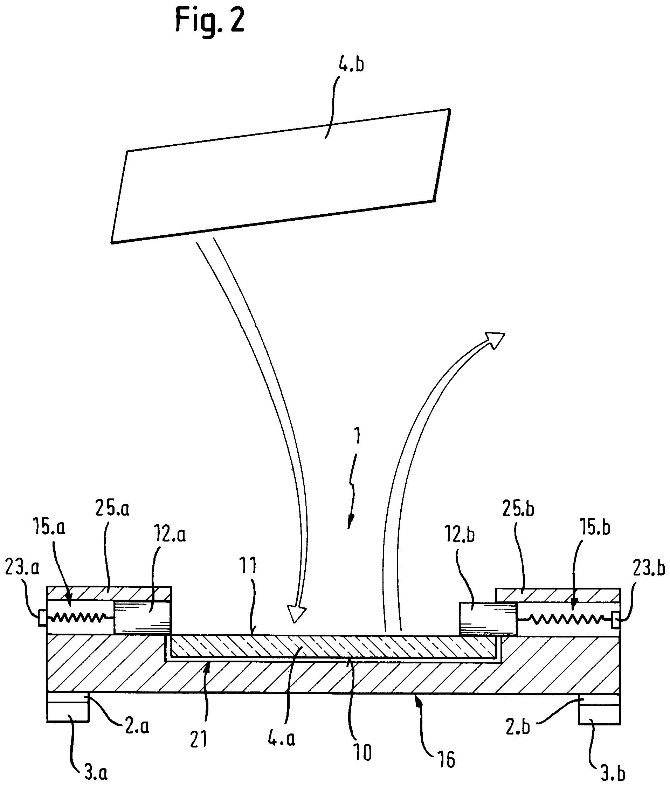

Fig. 2 zeigt eine weitere Ausführungsform der Personenwaage 1. Die Gewichtsermittlung erfolgt in den Kraftmesselementen 2a, 2b, die unabhängig von der Wiegeplatte 4.a in die Basiseinheiten 3a, 3b der Personenwaage 1 integriert sind. Die Wiegeplatte 4.a ist in der Regel transparent, kann aber auch gegen eine nicht-transparente Wiegeplatte ersetzt werden. Die Wiegeplatte 4.a der Personenwaage 1 kann verschiedene Formen besitzen. Die Form kann 2-dimensional ein, z.B. ein Rechteck mit geraden Kanten, ebenso kann sie 3-dimensional sein, z.B. ein Rechteck mit einer Schwalbenschwanzform in der Kante. In der vorliegenden Ausführungsform weist sie eine 2-dimensionale Form auf; sie ist rechteckig mit geraden Kanten. Die Wiegeplatte 4.a wird in eine U-förmige Struktur eingesetzt. An der Kopfseite der U-förmigen Struktur sind zwei Kugelschreiber-Mechanismen 15a, 15b mit je einem Knopf 23.a, 23.b und einem Gelenk 24a, 24b angebracht. An diesen Kugelschreiber-Mechanismen 15a, 15b befinden sich je eine axiale Fixierung 12a, 12b der Wiegeplatte 4.a. Durch Drücken der Knöpfe 23.a, 23.b der Kugelschreiber-Mechanismen 15a, 15b schieben diese die Fixierung 12a, 12b der Wiegeplatte 4 etwas nach vorn. Somit ist die Wiegeplatte 4.a gegen ein Herausrutschen gesichert und die Gelenke 24a, 24b in ihrer Bewegung blockiert. Die Kugelschreiber-Mechanismen 15a, 15b sind an den Seiten flächenbündig. Zur Entnahme der Wiegeplatte 4.a müssen die Kugelschreiber-Mechanismen 15a, 15b erneut gedrückt werden, die Fixierungen 12a, 12b werden zurückgezogen und die Gelenke 24a, 24b sind wieder in der Bewegung frei.

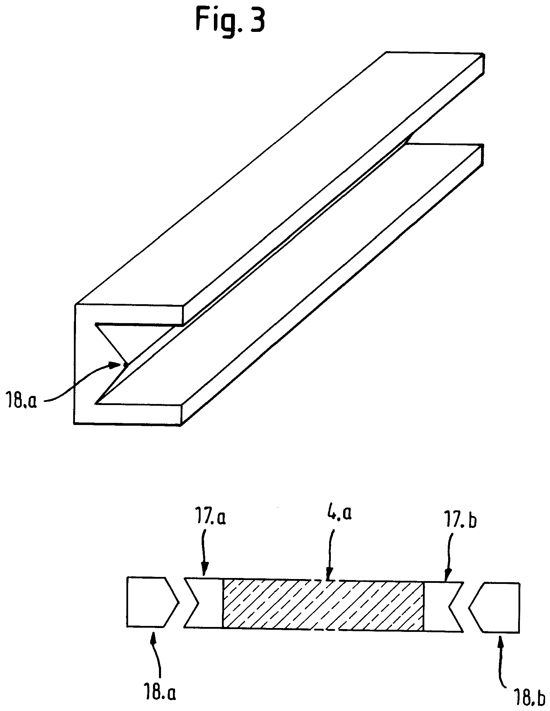

Fig. 3 zeigt eine weitere Ausführungsform der Befestigung der Wiegeplatte 4.a. An der Wiegeplatte 4.a ist seitlich eine Struktur 17.a, 17.b aufgebracht. Diese Struktur 17.a, 17.b kann z.B. ein aufgeklebtes Stück aus Kunststoff oder Metall sein. Diese Struktur 17.a, 17.b kann auch ein Teil der Wiegeplatte 4.a selbst sein, z.B. indem die Struktur 17.a, 17.b in die Wiegeplatte 4.a gefräst, geschnitten, gebohrt, geätzt, gesägt oder mittel Lasertechnik eingefügt wird. Die Wiegeplatte 4.a wird dann horizontal in eine entsprechend geformte Nut 18.a, 18.b geschoben und über eine Arretierung, in diesem Fall über eine Kugel, axial fixiert. Dies ermöglicht auf der einen Seite eine sichere Befestigung der Wiegeplatte 4.a mit der Basiseinheit 3, so dass die Gewichtsdaten zuverlässig und sicher ermittelt werden können und auf der anderen Seite gleichzeitig, dass die Personenwaage 1 gut transportiert werden kann, ohne dass die Wiegeplatte 4.a sich versehentlich aus der Arretierung löst. Gleichzeitig ermöglicht diese Befestigung aber auch einen einfachen und für den Anwender wenig aufwändigen Austausch der Wiegeplatte 4.a gegen eine Wiegeplatte 4.b.

Schließlich haben sich Mittel zum Kalibrieren der Waage 1 bewährt die gewährleisten, dass eine Gewichtsmessung nach dem Wechseln einer Wiegeplatte 4.a eggen eine Wiegeplatte 4.b zuverlässig und reliabel durchgeführt werden kann. Dabei kann in einem Taraprogramm nach Einsetzen der Wiegeplatte 4.b sichergestellt werden, dass die Personenwaage 1 bei nicht belasteter Wiegeplatte 4.b tatsächlich ein Gewicht von 0 Kilogramm ermittelt und anzeigt. Wird dieses Programm durchlaufen und abgeschlossen, kann die Gewichtsermittlung beginnen. Dieses Programm kann beliebig oft durchlaufen werden, sollte aber zumindest nach jedem Wechsel der Wiegeplatte 4.a gegen 4.b mindestens einmal erfolgen.

Die erfindungsgemäße Personenwaage 1 weist gegenüber herkömmlichen Personenwaagen erheblich verbesserte Gestaltungsmöglichkeiten auf und ist darüber hinaus insbesondere auch in hygienischer Hinsicht äußerst vorteilhaft.

### BEZUGSZEICHENLISTE

- 1: Personenwaage
- 2.a; 2.b; 2.c; 2.d: Kraftmesselement
- 3.a; 3.b; 3.c; 3.d: Basiseinheit
- 4.a; 4.b: Erste Wiegeplatte; weitere Wiegeplatte
- 5: Anzeige
- 6: Speichereinheit
- 7: Computer
- 8: Display
- 9: Wechselbauteil Mikrochip
- 10: Von dem Benutzer abgewandte Seite
- 11: Dem Benutzer zugewandte Seite
- 12.a; 12.b: Axiale Fixierung
- 13: Waagenoberfläche
- 14: Versenkbarer Knopf
- 15.a; 15.b: Kugelschreiber-Mechanismus
- 16: Tragstruktur
- 17.a; 17.b: Seitliche Struktur
- 18.a; 18.b: Nut
- 21: U-förmige Struktur
- 23.a; 23.b: Knopf
- 24.a; 24.b: Gelenk
- 25.a; 25.b: Halterung

## Patentansprüche

1. Personenwaage (1), umfassend wenigstens eine Basiseinheit (3), ein in der Basiseinheit (3) integrierbares Kraftmesselement (2), eine auf das Kräftmesselement (2) wirkende Wiegeplatte (4.a), sowie eine Anzeige (5) zum Anzeigen zumindest des von dem Kraftmesselement (2) gemessenen Gewichts, wobei wenigstens eine Halterung (25) zur Befestigung der Wiegeplatte (4.a) mit der Basiseinheit (3) vorgesehen ist, mittels derer die Wiegeplatte (4.a) durch einen Anwender entnehmbar und gegen eine andere Wiegeplatte (4.b) austauschbar ist, **dadurch gekennzeichnet, dass** die Halterung (25) zur Sicherung der Wiegeplatte (4.a) gegen ein Herausrutschen zwei Kugelschreiber-Mechanismen (15a, 15b) mit je einem Knopf (23.a, 23.b) und je einer axialen verschiebbaren Fixierung (12a, 12b) der Wiegeplatte (4.a) enthält und/oder eine an der Wiegeplatte (4.a) angebrachte seitliche Struktur (17.a, 17.b) enthält, über die die Wiegeplatte (4.a) in eine entsprechend geformte Nut (18a. 18b) horizontal geschoben und über eine Arretierung axial fixiert ist.

2. Personenwaage (1) nach Anspruch 1, **dadurch gekennzeichnet, dass** wenigstens zwei, vorzugsweise vier, Basiseinheiten (3a, 3b, ...) vorgesehen sind.

3. Personenwaage (1) nach Anspruch 1 oder 2, **dadurch gekennzeichnet, dass** eine Tragstruktur (16) zur Halterung der Basiseinheit/en (3; 3a, 3b, ...) vorgesehen ist.

4. Personenwaage (1) nach einem der Ansprüche 1 bis 3, **dadurch gekennzeichnet, dass** die Personenwaage (1) eine Speichereinheit (6) aufweist, die Informationen speichert, mittels derer eine definierte Wiegeplatte (4.a; 4.b) einer definierten Person zugeordnet werden kann.

5. Personenwaage (1) nach einem der vorherigen Ansprüche, **dadurch gekennzeichnet, dass** die Personenwaage (1) eine elektronische Speichereinheit (6) zum Speichern von Benutzerdaten, insbesondre Gewichtsdaten, aufweist.

6. Personenwaage (1) nach Anspruch 4 oder 5, **dadurch gekennzeichnet, dass** die Speichereinheit (6) eine Datenschnittstelle zur Datenübertragung an einen zugeordneten Computer (7) aufweist.

7. Personenwaage (1) nach Anspruch 6, **dadurch gekennzeichnet, dass** die Datenübertragung zum Computer (7) mittels Funk und/oder Infrarotübertragung und/oder Übertragungskabel herstellbar ist.

8. Personenwaage (1) nach einem der Ansprüche 4 bis 7, **dadurch gekennzeichnet, dass** die Personenwaage (1) eine biometrische Zugangskontrolle aufweist, die sicherstellt, dass erst nach Erfüllen eines oder mehrerer Zugangskriterien für einen Anwender ein definierter Speicherbereich und/oder ein definiertes Programm der Personenwaage (1) zugänglich ist.

9. Personenwaage (1) nach Anspruch 8, **dadurch gekennzeichnet, dass** die biometrische Zugangskontrolle über einen Vergleich der Stimme und/oder der Iris und/oder des Fuß- und/oder Fingerabdruckes ausführbar ist.

10. Personenwaage (1) nach einem der vorhergehenden Ansprüche, **dadurch gekennzeichnet, dass** die Wiegeplatte (4.a; 4.b) wenigstens ein, vorzugsweise auswechselbares, Display (8) aufweist.

11. Personenwaage (1) nach einem der vorhergehenden Ansprüche, **dadurch gekennzeichnet, dass** die Wiegeplatte (4.a; 4.b) wenigstens ein Wechselbauteil (9) wie einen Mikrochip und/oder die Speichereinheit (6) umfasst, welche/r (6; 9) aus der Wiegeplatte (4) entnehmbar ausgebildet ist.

12. Personenwaage (1) nach einem der vorhergehenden Ansprüche, **dadurch gekennzeichnet, dass** die Wiegeplatte (4.a; 4.b) eine antibakterielle Beschichtung aufweist.

13. Personenwaage (1) nach einem der vorhergehenden Ansprüche, **dadurch gekennzeichnet, dass** die Personenwaage (1) eine Körperfettwaage ist.

## Claims

1. Personal scales (1) comprising at least one base unit (3), a force measuring element (2) that can be integrated into the base unit (3), a weighing plate (4.a) acting on the force measuring element (2), and a display (5) for displaying at least the weight measured by the force measuring element (2), wherein at least one bracket (24) is provided for attaching the weighing plate (4.a) to the base unit (3), by means of which the weighing plate (4.a) can be removed by a user and exchanged for a different weighing plate (4.b), **characterised in that** the bracket (25) contains, in order to secure the weighing plate (4.a) from slipping out, two ballpoint mechanisms (15a, 15b), each with a stud (23.a, 23.b) and each with an axial movable fixing (12a, 12b) of the weighing plate (4.a) and/or contains a lateral structure (17.a, 17.b) attached to the weighing plate (4.a), via which the weighing plate (4.a) is pushed horizontally into a correspondingly formed groove (18a, 18b) and is axially fixed by a locking mechanism.

2. Personal scales (1) according to claim 1, **characterised in that** at least two, preferably four, base units (3a, 3b , ...) are provided.

3. Personal scales (1) according to claim 1 or 2, **characterised in that** a support structure (16) for holding the base unit/s (3; 3a, 3b, ...) is provided.

4. Personal scales (1) according to one of claims 1 to 3, **characterised in that** the personal scales (1) have a memory unit (6), which stores information by which a defined weighing plate (4.a; 4.b) can be assigned to a defined person.

5. Personal scales (1) according to one of the preceding claims, **characterised in that** the personal scales (1) have an electronic memory unit (6) for storing user data, in particular weight data.

6. Personal scales (1) according to claim 4 or 5, **characterised in that** the memory unit (6) has a data interface for data transfer to an associated computer (7).

7. Personal scales (1) according to claim 6, **characterised in that** the data transmission to the computer (7) can be produced by means of radio and/or infrared transmission and/or transmission cables.

8. Personal scales (1) according to one of claims 4 to 7, **characterised in that** the personal scales (1) have a biometric access control which ensures that only after fulfilling one or more access criteria for a user is a defined memory area and/or a defined program of the personal scales (1) accessible.

9. Personal scales (1) according to claim 8, **characterised in that** the biometric access control can be executed by way of a comparison of the voice and/or iris and/or footprint and/or fingerprint.

10. Personal scales (1) according to one of the preceding claims, **characterised in that** the weighing plate (4.a; 4.b) has at least one, preferably interchangeable, display (8).

11. Personal scales (1) according to one of the preceding claims, **characterised in that** the weighing plate (4.a; 4.b) comprises at least one replacement component (9) such as a microchip and/or the memory unit (6) that (6; 9) is designed to be removable from the weighing plate (4).

12. Personal scales (1) according to one of the preceding claims, **characterised in that** the weighing plate (4.a; 4.b) has an antibacterial coating.

13. Personal scales (1) according to one of the preceding claims, **characterised in that** the personal scales (1) are body fat scales.

## Revendications

1. Pèse-personne (1), comprenant au moins une unité de base (3), un élément de mesure de force (2) pouvant être intégré dans l'unité de base (3), un plateau de pesée (4.a) agissant sur l'élément de mesure de force (2), ainsi qu'un affichage (5) pour afficher au moins le poids mesuré par l'élément de mesure de force (2), dans lequel au moins un support (25) est prévu pour la fixation du plateau de pesée (4.a) avec l'unité de base (3), support au moyen duquel le plateau de pesée (4.a) peut être retiré par un usager et peut être remplacé par un autre plateau de pesée (4.b), **caractérisé en ce que** le support (25) contient pour la protection du plateau de pesée (4.a) contre un glissement deux mécanismes de stylo à bille (15a, 15b) avec respectivement une tête (23.a, 23.b) et respectivement une fixation mobile axiale (12a, 12b) du plateau de pesée (4.a) et/ou contient une structure latérale (17.a, 17.b) installée au niveau du plateau de pesée (4.a) par l'intermédiaire de laquelle le plateau de pesée (4.a) coulisse horizontalement dans une rainure de forme correspondante (18.a, 18.b) et est fixé axialement par l'intermédiaire d'une butée.

2. Pèse-personne (1) selon la revendication 1, **caractérisé en ce que**, au moins deux, de préférence quatre, unités de base (3a, 3b, ...) sont prévues.

3. Pèse-personne (1) selon la revendication 1 ou 2, **caractérisé en ce qu'**une structure porteuse (16) pour le support de l'unité de base/des unités de base (3 ; 3a, 3b, ...) est prévue.

4. Pèse-personne (1) selon l'une des revendications 1 à 3, **caractérisé en ce que** le pèse-personne (1) présente une unité d'enregistrement (6), qui enregistre des informations au moyen desquelles un plateau de pesée défini (4.a ; 4.b) peut être associé à une personne définie.

5. Pèse-personne (1) selon l'une des revendications précédentes, **caractérisé en ce que** le pèse-personne (1) présente une unité d'enregistrement électronique (6) pour l'enregistrement de données d'utilisateur, en particulier des données de poids.

6. Pèse-personne (1) selon la revendication 4 ou 5, **caractérisé en ce que** l'unité d'enregistrement (6) présente une interface de données pour le transfert de données à un ordinateur associé (7).

7. Pèse-personne (1) selon la revendication 6, **caractérisé en ce que** le transfert de données à l'ordinateur (7) peut être généré au moyen d'un transfert radio et/ou infrarouge et/ou d'un câble de transfert.

8. Pèse-personne (1) selon l'une des revendications 4 à 7, **caractérisé en ce que** le pèse-personne (1) présente un contrôle d'accès biométrique, qui garantit que ce n'est qu'après qu'un ou plusieurs critères d'accès pour un usager ont été remplis qu'une zone de mémoire définie et/ou qu'un programme défini du pèse-personne (1) sont accessibles.

9. Pèse-personne (1) selon la revendication 8, **caractérisé en ce que** le contrôle d'accès biométrique est réalisable par l'intermédiaire d'une comparaison des voix et/ou de l'iris et/ou de l'empreinte des pieds et/ou des doigts.

10. Pèse-personne (1) selon l'une des revendications précédentes, **caractérisé en ce que** le plateau de pesée (4.a ; 4.b) présente au moins un écran (8), de préférence remplaçable.

11. Pèse-personne (1) selon l'une des revendications précédentes, **caractérisé en ce que** le plateau de pesée (4.a ; 4.b) comprend au moins un composant à remplacer (9) comme une micropuce et/ou l'unité d'enregistrement (6), qui (6 ; 9) est réalisé pour pouvoir être retiré du plateau de pesée (4).

12. Pèse-personne (1) selon l'une des revendications précédentes, **caractérisé en ce que** le plateau de pesée (4.a ; 4.b) présente un revêtement antibactérien.

13. Pèse-personne (1) selon l'une des revendications précédentes, **caractérisé en ce que** le pèse-personne (1) est un analyseur de graisse corporelle.
